(19) Europäisches Patentamt
European Patent Office
Office européen des brevets

(11) Veröffentlichungsnummer: **0 005 121**
**B1**

(12) # EUROPÄISCHE PATENTSCHRIFT

(45) Veröffentlichungstag der Patentschrift: **13.10.82**

(51) Int. Cl.³: **A 61 K 31/425**, A 61 K 9/48

(21) Anmeldenummer: **79810032.7**

(22) Anmeldetag: **09.04.79**
**Die Akte enthält technische Angaben, die nach dem Eingang der Anmeldung eingereicht wurden und die nicht in dieser Patentschrift enthalten sind.**

(54) **Verfahren zum Stabilisieren von 4-Methyl-5-(2'-chloraethyl)-thiazol.**

(30) Priorität: **25.04.78 AT 2961/78**

(43) Veröffentlichungstag der Anmeldung: **31.10.79 (Patentblatt 79/22)**

(45) Bekanntmachung des Hinweises auf die Patenterteilung: **13.10.82 Patentblatt 82/41**

(84) Benannte Vertragsstaaten: **BE CH DE FR GB IT LU NL SE**

(56) Entgegenhaltungen:
**BE A 693 977**
**DE A 2 631 281**
**FR A 1 416 304**
**FR A 1 536 904**
**FR A 2 206 943**
**FR A 2 262 527**
**FR A 2 321 892**
**FR A 2 365 338**
**GB A 931 149**
**US A 2 844 512**
**US A 3 146 167**

(73) Patentinhaber: **PHARMACEUTICAL LICENCES COMPANY LTD.**
**7, Chemin de Trembley**
**CH-1197 Prangins VD (CH)**

(72) Erfinder: **Feisch, Horst**
**Schlossberg 25**
**A-6391 Fieberbrunn (AT)**
Erfinder: **Hantich, Gerhard**
**Stockerdörfl 25**
**A-6370 Kitzbühel (AT)**

(74) Vertreter: **Meyer, Hans**
**15 rue Viollier**
**CH-1207 Genève (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

EP 0 005 121 B1

Verfahren zum Stabilisieren von 4-Methyl-5-(2'-chloraethyl)-thiazol

Die Erfindung bezieht sich auf ein Verfahren zum Stabilisieren von 4-Methyl-5-(2'-chloräthyl)-thiazol mittels nicht toxischen Glyceriden als Trägersubstanz zum Füllen von Kapseln.

Es ist bekannt, dass 4-Methyl-5-(2'-Chloräthyl)-thiazol der Formel

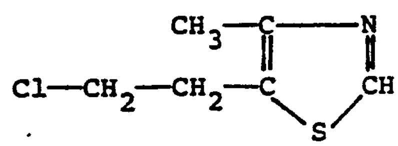

(im folgenden als CMT abgekürzt) sedativ und anticonvulsiv wirksam ist. Die CMT-Base ist aber ihrer chemischen Struktur nach instabil. Es wurde bereits erkannt (BE-PS 693 977) dass es, insbesondere bei längerer Lagerung und bei höheren Temperaturen, zu Zersetzungsreaktionen kommt, und zwar zu einer HCl-Abspaltung. Die entstehenden Zersetzungsprodukte haben nicht mehr die gleichen pharmakologischen Wirkungen wie die CMT-Base.

Ein weiterer Nachteil besteht darin, dass das CMT üblicherweise, insbesondere bei Infusionslösungen und Injektionslösungen, in Form eines Aethandisulfonates verwendet wird. Für perorale Formen bringt dies jedoch den Nachteil eines unangenehmen Geschmackes und Geruches mit sich, der von kleineren Mengen freier Base herrührt und der bisher kaum erfolgreich maskiert werden konnte.

Die vorliegende Erfindung hat die Aufgabe, das eingangs genannte Verfahren derart zu verbessern, dass die Stabilität der CMT-Base ausreichend gesteigert wird, um damit gefüllte Kapseln bei etwa Raumtemperatur bis zu sechs oder noch mehr Jahren haltbar zu machen, ohne dass die Wirksamkeit, der Geruch oder der Geschmack beeinträchtigt werden. Diese Aufgabe wird durch die Massnahmen gelöst, die im kennzeichnenden Teil des Anspruchs 1 definiert sind.

Zwar ist es an sich bekannt (Patentschriften GB 931.149, US 3.146.167, FR 1.416.304, FR 1.536.904, FR 2.262.527, FR 2.321.892, FR 2.206.943) derartige Triglyceride den verschiedensten pharmazeutischen Wirkstoffen beizumischen, wobei die zitierte GB-PS auch nebenbei einen möglichen Stabilisierungseffekt andeutet. Keine der genannten Patentschriften befasst sich jedoch mit den speziellen Problemen der CMT-Base, die nicht einmal andeutungsweise erwähnt wird.

Anderseits wurde in der bereits genannten BE-PS 693.977 schon vorgeschlagen, das CMT mit einem nichtpolaren nichttoxischen Träger zu vermischen, der aus einem oder mehreren tierischen oder pflanzlichen Oelen oder Fetten, insbesondere Erdnussöl besteht, oder aus einer oder mehreren höheren Fettsäuren, wobei letztere Verbindungen mit 14 bis 22 Kohlenstoffatome, sind. Damit lässt sich die Zersetzung der CMT-Base bremsen, so dass die erhaltenen Präparate über einige Zeit lagerfähig sind. Der unangenehme schwefelige Geruch und der bittere Geschmack haftet aber den Präparaten nach wie vor an. Der genannte Stand der Technik löst also das Problem der Stabilisierung der CMT-Base nicht.

Untersuchungen der Anmelderin haben nunmehr ergeben, dass neben der eingangs erwähnten HCl-Abspaltung noch weitere Reaktionen zur Zersetzung der CMT-Base führen. Wird nämlich frisch destillierte CMT-Base einige Tage bei erhöhter Temperatur gehalten und die Zersetzungsprodukte in Form des ausgefallenen braunen Niederschlages mit Benzol gewaschen und untersucht, so ergibt sich, dass die Menge an Gesamtchlorid wesentlich höher ist als die Summe von freiem Chlorid und Chlorid von okkludierter CMT-Base und CMT-HCl. Der Chloridüberschuss muss demnach an Zersetzungsprodukte der CMT-Base gebunden sein.

Ausserdem haben die Untersuchungen gezeigt, dass neben der HCl-Abspaltung für ein Gemisch von CMT-Base und einer Trägersubstanz letztere anfällig für Autoxydationsreaktionen ist, wenn die Trägersubstanz reaktive Gruppen wie z.B. freie Säuren oder Alkoholkomponenten oder olefinische Doppelbindungen enthält, wie dies bei pflanzlichen und tierischen Oelen oder Fetten der Fall ist. Neben der HCl-Abspaltung treten radikalische Zersetzungsmechanismen der CMT-Base auf Basis von Autoxydationsreaktionen auf. Diese zusätzlich zur HCl-Abspaltung auftretende Zersetzung tritt umso mehr in Erscheinung, je mehr die verwendete Trägersubstanz anfällig für Autoxydationsreaktionen ist. Aus diesem Grund erfolgt im Sinne der Erfindung die Wahl einer physiologisch unbedenklichen Trägersubstanz, welche neben einer Stabilisatorwirkung hinsichtlich der HCl-Abspaltung unempfindlich gegen Luftsauerstoff ist und dadurch keine Zersetzung der CMT-Base durch Autoxydationsreaktionen induziert. Trägersubstanzen, die tierische oder pflanzliche Oele oder Fette enthalten, kommen daher nicht in Frage, weil diese durch ihren Gehalt an ungesättigten Fettsäuren ausserordentlich autoxydationsgefährdet sind. Durch nachträgliche Massnahmen, z.B. Hydrierung, lässt sich der Anteil an Doppelbindungen zwar senken, aber nicht in dem Masse, dass das Autoxydationsvermögen massgeblich gemindert wird.

Triglyceride der erfindungsgemässen Art sowie deren Gemische sind physiologisch unbedenklich, da sie nach dem Fettsäurezyklus abgebaut werden. Solche Triglyceride oder Triglyceridgemische auf Basis gesättigter Fettsäuren mit mittlerer Kettenlänge ($C_8$ bis $C_{12}$) lassen sich auf synthetischem Wege durch Verestern von Glycerin mit Fettsäuren leicht herstellen, z.B. als gesättigte n-Alkansäuren mittlerer Kettenlänge mit einer geradzahligen Kohlenstoffanzahl (gesättigte pflanzliche oder tierische Fettsäuren

mittlerer Kettenlänge). Die verwendeten Fettsäuren können auf synthetischem Wege gewonnen werden oder durch Verseifen natürlicher Fette oder Oele, Hydrierung und Reinigung. Derartige Triglyceride oder Triglyceridgemische enthalten keine Doppelbindungen mehr (Jodzahl kleiner als 1) und sind sehr gut haltbar. Die Verwendung von Glycerin als Alkoholkomponente und gesättigten n-Alkansäuren mittlerer Kettenlänge mit einer geradzahligen Kohlenstoffanzahl als Säurekomponente erklärt sich aus der Notwendigkeit einer guten physiologischen Verträglichkeit.

Gemäss dem erfindungsgemässen Verfahrens wird als Trägersubstanz ein doppelbindungsfreies Triglyceridgemisch auf Basis gesättigter mittlerer Fettsäuren der Kettenlänge $C_8$ bis $C_{12}$, insbesondere mit gerader Kohlenstoffatomanzahl, eingesetzt, dessen Jodzahl kleiner als 1 ist, vorzugsweise ein schwermetallionenarmes Neutralöl. Derartige Triglyceridgemische sind sehr gut haltbar. An sich bekannte Triglyceridgemische gesättigter Pflanzenfettsäuren mit einer Kettenlänge von $C_8$ bis $C_{12}$ haben einen Gehalt an $C_6$ Kettenlängen von maximal 3 %, was zu vernachlässigen ist. Ihre Jodzahl liegt unter 1 und der Schwermetallspurenwert unter 0,001 %. Die Säurezahl liegt unter 0,1. Diese Daten sind in Bezug auf Unempfindlichkeit gegen Autoxydation besonders günstig.

Durch Versuche der Patentinhaberin hat sich ferner herausgestellt, dass die erwähnte Autoxydation nicht nur durch geeignete Wahl der Trägersubstanz verringert bzw. vermieden werden kann, sondern auch dadurch, dass im Rahmen der Erfindung die Vermischung des 4-Methyl-5-(2'-chloräthyl)-thiazol mit der Trägersubstanz unter Ausschluss von Luftsauerstoff, vorzugsweise in einer Inertgasatmosphäre, insbesondere Stickstoffatmosphäre erfolgt. Vorzugsweise wird hiebei erfindungsgemäss so vorgegangen, dass die erhaltene Mischung weiter bis zur luftblasenfreien Abfüllung in Weichgelatinekapseln unter Inertgasatmosphäre, insbesondere Stickstoffatmosphäre, gehalten wird. Gegebenenfalls können jedoch auch andere Inertgase, wie Krypton, Argon oder Neon Verwendung finden, jedoch ist Stickstoff aus Preisgründen am günstigsten.

Die Begasung mit Inertgas verhindert bzw. vermindert Zersetzungsreaktionen, welche durch die Anwesenheit von Luftsauerstoff induziert bzw. beschleunigt werden. Wie bereits erwähnt, werden derartige Autoxydationsreaktionen meist durch die Anwesenheit reaktiver Gruppen (z.B. olefinischer Doppelbindungen, freier Säuren) oder durch Schwermetallionen beschleunigt. Da nun einerseits die Trägersubstanz so gewählt ist, dass sie im Hinblick auf derartige Reaktionen möglichst neutral reagiert (insbesondere Jodzahl kleiner als 1, Säurezahl kleiner als 0,1, Schwermetallionenspuren kleiner als 0,001 %), anderseits der Zutritt von Luftsauerstoff sowohl bei der Mischung als auch bei der anschliessenden Lagerung und Abfüllung des Gemisches vermieden wird, sind solche Autoxydationsreaktionen derart vermindert, dass die erhaltene Mischung über mehrere Jahre hindurch lagerfähig ist. Die luftblasenfreie Abfüllung des Gemisches aus CMT-Base und Trägersubstanz in Weichgelatinekapseln ist beispielsweise nach dem Scherer-Verfahren leicht möglich. Zweckmässig enthalten die Weichgelatinekapseln zur Vermeidung von Versprödung Weichmacher wie Sorbit oder Glycerin. Eine besonders gute Dichtheit, auch in Bezug auf Geruchsdichtheit, der Gelatinekapseln konnte durch einen Glyceringehalt von 10 bis 20 Gew.-% und einen Sorbitgehalt von 10 bis 20 Gew.-% erreicht werden.

Unter den im Rahmen der Erfindung besonders geeigneten Triglycerid gemischen sind jene besonders geeignet, die folgende Fettsäurenverteilung haben :

| | | | | |
|---|---|---|---|---|
| Capronsäure | ($C_6$) | 3 % max. | 3 % max. | 3 % max. |
| Caprylsäure | ($C_8$) | 65-80 % | 50-65 % | 65-80 % |
| Caprinsäure | ($C_{10}$) | 15-30 % | 30-45 % | 15-30 % |
| Laurinsäure | ($C_{12}$) | 3 % max. | 5 % max. | 3 % max. |
| Linolsäure | ($C_{18}$) | — | — | — |

Durch Versuche der Anmelderin hat sich gezeigt, dass als Trägersubstanz ausser diesen Verbindungen auch Glycerintricaprinat oder Glycerintrilaurat eingesetzt werden können.

Will man für spezielle pharmazeutische Anwendungen eine gesteuerte Freigabe des Wirkstoffes im Darmtrakt erreichen, so ist es zweckmässig, die Kapsel mit einem magensaftresistenten darmlöslichen Ueberzug aus einem Polymethacrylsäure-Derivat oder einem Cellulosederivat, insbesondere aus Hydroxypropylmethylcellulosephthalat und Dibutylphthalat zu überziehen.

Das Mischungsverhältnis von CMT-Base und Trägersubstanz kann im Rahmen der Erfindung innerhalb weiter Grenzen variieren. Im allgemeinen ist die Mindestkonzentration an Trägersubstanz durch den zu erreichenden Stabilitätseffekt begrenzt. Der Mindestgehalt an Trägersubstanz des Gemisches liegt bei etwa 20 Gew.-%. Bei Unterschreitung dieser Grenze ist der Stabilisierungseffekt nicht mehr ausreichend. Der Maximalanteil an Trägersubstanz ist im allgemeinen nicht begrenzt, ausser für oral applizierbare Kapseln. In diesem Fall wird nämlich bei einem Gehalt der Mischung über 80 % an Trägersubstanz das Gesamtvolumen der Kapsel so hoch, dass eine orale Applizierbarkeit in Frage gestellt ist.

Die Menge des als Einzeldosis in der Gelatinekapsel eingeschlossenen Gemisches von CMT-Base und Trägersubstanz hängt vom speziellen Anwendungsfall ab. Im allgemeinen werden Mengen von 300 bis 1 000 mg als günstigste Werte anzusehen sein.

Durch die folgenden Beispiele wird die Erfindung noch näher erläutert.

Beispiel 1

Ein 10 Liter Edelstahlrührkessel in Vakuumausführung wird mit 4,0 kg Neutralöl Miglyol 812 gefüllt und nach Entfernung der Luft durch Anlegen eines Vakuums (10 min, 133 Pa) mit technisch reinem Stickstoff begast. Der Stickstoff wird dabei mit einem Verteiler direkt in das Neutralöl geleitet. Unter weitere Stickstoffbegasung wird nun unter Rühren 4,0 kg frisch destillierte $O_2$-freie CMT-Base mit einem Chloridgehalt von etwa 0,01 % kontinuierlich langsam zugeführt, und zwar mit 200 ml/min. Das Gemisch wird nach weiteren 10 min Rührzeit in Edelstahltransportbehälter gefüllt. Anschliessend wird die Mischung luftblasenfrei in magensaftlösliche Weichgelatinekapseln abgefüllt. Die Füllmenge pro Kapsel beträgt 500 mg Gemisch.

Beispiel 2

Ein 5 Liter evakuierbarer (Rundkolben) Glasrührkessel wird mit 1,5 kg Tricaprin (Glycerin-tricaprinat) gefüllt und nach Entfermung der Luft durch Anlegen eines Vakuums (10 min, 133 Pa) mit technisch reinem Argon begast. Das Argon wird dabei mittels eines Verteilers direkt in die Tricaprin-Lösung geleitet. Unter weiterer Argon-Bagasung wird nun unter Rühren 1,0 kg frisch destillierte $O_2$-freie CMT-Base mit einem Chloridgehalt von etwa 0,01 % während einer Zeitdauer von 10 min kontinuierlich zugetropft und das Gemisch in eine Argon-begaste 3-Liter-Glasflasche gefüllt. Anschliessend wird die Mischung luftblasenfrei in magensaftresistente, darmlösliche Weichgelatinekapseln abgefüllt, von denen jede 600 mg Gemisch enthält.

Beispiel 3

Ein 10 Liter Edelstahlrührkessel in Vakuumausführung wird mit 4,0 kg Tricaprylin (Glycerin-tricaprylat) gefüllt und gemäss Beispiel 1 weiterbehandelt.

Beispiel 4

Ein 10 Liter Edelstahlrührkessel in Vakuumausführung wird mit 2,0 kg Trilaurin (Glycerin-trilaurat) und 2,0 kg Tricaprin (Glycerin-tricaprinat) gefüllt und gemäss Beispiel 1 weiterbehandelt.

Beispiel 5

Ein 5 Liter evakuierbarer (Rundkolben) Glasrührkessel wird mit 1,5 kg Neutralöl in Form eines Triglyceridgemisches gesättigter Fettsäuren gefüllt und gemäss Beispiel 2 weiterbehandelt.

Ferner hat die Patentinhaberin Versuche zum Nachweis dafür abgestellt, dass zusätzlich zur HCl-Abspaltung noch andere Reaktionen zur Zersetzung der CMT-Base führen können.

Der Mechanismus der HCl-Abspaltung ist durch die folgende Formel gegeben :

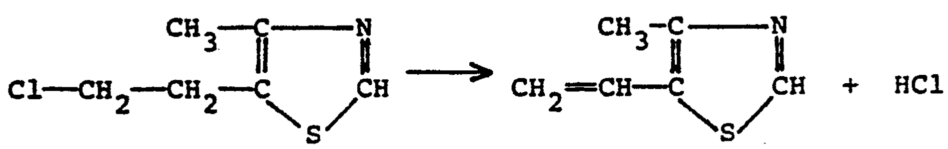

Wenn nur die HCl-Abspaltung zur Zersetzung der CMT-Base führt, dürfen keine Reaktionsprodukte, die gebundenes Chlorid enthalten, gefunden werden.

Es wurde nun frisch destillierte Clomethiazol-Base 7 Tage bei 70 °C gehalten, der ausgefallene braune Niederschlag (Zersetzungsprodukte) mit Benzol gewaschen und untersucht.

Die mit dem Niederschlag durchgeführten Bestimmungen des Gesamtchlorids, des freien Chlorids, gaschromatographische Bestimmung von okkludierte CMT-Base + CMT-HCl und $^1$H-NMR-spektroskopische Untersuchungen brachten folgendes Ergebnis :

Die Menge an bestimmtem Gesamtchlorid liegt um 17 % höher als die Summe von freiem Chlorid und Chlorid von okkludierter CMT-Base und CMT-HCl. Dieser 17 % Chloridüberschuss muss demnach an Zersetzungsprodukte der CMT-Base gebunden sein. Die H-NMR spektroskopischen Untersuchungen des Niederschlages erhärten obiges Ergebnis durch das Auftreten zusätzlicher Signale von aliphatischen Protonen, die den möglichen Zersetzungsprodukten der HCl-Abspaltung nicht zugeordnet werden konnten.

Es wurden 2 Mischungen von CMT-Base und Neutralöl hergestellt.

Mischung I : 83,5 Gew.-% CMT-Base,
16,5 Gew.-% Neutralöl.

Mischung II : 31,2 Gew.-% CMT-Base, 68,8 Gew.-% Neutralöl.

Versuchsanordnung 1 : Mischung I und II wurde in einem Glaskolben bei 70 °C unter Stickstoffatmosphäre 100 und 170 h lang erhitzt und die Zersetzung der CMT-Base bestimmt.

| | Zersetzung von CMT-Base in % | |
|---|---|---|
| | 100 Stunden | 170 Stunden |
| Mischung I | 3,5 | 4,9 |
| Mischung II | < 0,5 % | < 0,5 % |

Versuchsanordnung 2 : Mischung I und II wurden in einem Glaskolben unter Luftatmosphäre bei 70 °C 170 h lang erhitzt und die Zersetzung der CMT-Base bestimmt.
Zersetzung der CMT-Base in % nach 170 Stunden

| | |
|---|---|
| Mischung I | 14,1 % |
| Mischung II | 3,7 % |

Eine vergleichende Betrachtung der beiden Versuchsanordnungen ergibt, dass der Zusatz von Neutralöl in Form eines Triglyceridgemisches gesättigter Fettsäuren die Zersetzung der CMT-Base vermindert. Mit zunehmender Neutralölkonzentration steigt diese Stabilisatorwirkung.

Durch Ausschluss des Luftsauerstoffes kann eine wesentliche Erhöhung der Stabilisatorwirkung erreicht werden. Dies beweist, dass Luftsauerstoff wesentlich an der Zersetzung der CMT-Base beteiligt ist, und Mechanismen der Autoxydation wirksam werden. Wäre die HCl-Abspaltung die einzig stattfindende Zersetzungsreaktion, müssten unter Luft- und Stickstoffatmosphäre die erhaltenen Werte übereinstimmen.

Auf Grund der ausgezeichneten Ergebnisse der Versuchsanordnung 1 wurden Weichgelatinekapseln (Zusammensetzung : 67 Gew.-% Gelatine, 19 Gew.-% Glycerin, 14 Gew.-% Sorbit) mit 330 mg Gemisch (192 mg CMT-Base, 138 mg Neutralöl) unter Stickstoffspülung gefüllt und 16 Monate lang bei 20 °C, 30 °C, 40 °C und 50 °C gelagert. Anschliessend wurde die Zersetzung der CMT-Base bestimmt.

Die Zersetzung der CMT-Base wurde in sämtlichen Fällen über die gaschromatographische Bestimmung der noch unzersetzten Base berechnet. Dies hat gegenüber dem Verfahren der Messung des freien Chlorids den Vorteil, dass auch möglich Zersetzungsreaktionen, bei denen das Chlorid am Zersetzungsprodukt gebunden bleibt, berücksichtigt werden. Vergleichende Bestimmungen der Zersetzung über das freie Chlorid zeigten, dass die gaschromatographisch bestimmten Zersetzungswerte etwas höher lagen.

Ergebnis :

| Temperatur | Zersetzung der CMT-Base in % |
|---|---|
| 20 °C | 3,1 % |
| 30 °C | 8,3 % |
| 40 °C | 9,6 % |
| 50 °C | 22,9 % |

Mit den erhaltenen Werten erfolgte nach den Gesetzen der chemischen Reaktionskinetik unter Zuhilfenahme dazu veröffentlichter Literatur eine Haltbarkeitsvorausberechnung. Bei Raumtemperatur ist mit einer Haltbarkeit von vier Jahren zu rechnen. Sämtliche Kapseln waren nach der Temperaturbehandlung absolut geruchsdicht.

Die chemische Stabilität pharmazeutischer Produkte ist ein Hauptkriterium für die Therapiesicherheit und für die ökonomische Arzneimittelherstellung sowie deren Vertrieb, und somit eine grundlegende Forderung der Zulasungsbehörde. Die nachweisliche Verlängerung der Haltbarkeit stellt folglich eine wesentliche Verbesserung des Arzneimittelwertes dar.

**Ansprüche**

1. Verfahren zum Stabilisieren von 4-Methyl-5-(2'-chloraethyl)-thiazol mittels nicht toxischen Glyceriden als Trägersubstanz zum Füllen von Kapseln, dadurch gekennzeichnet, dass man Glyceride von gesättigten Fettsäuren mit 8 bis 12 C-Atomen in einer Menge von 20 bis 80 Gew.% bezogen auf die gesamte Mischung verwendet und dass das Vermischen in Anwesenheit von weniger als 0,001 % Schwermetallkationen und unter Ausschluss von Luftsauerstoff in einer Inertgasatmosphäre, insbesondere unter Stickstoff erfolgt.

2. Verfahren nach Anspruch 1, dadurch gekennzeichnet, dass man als Glyceride Glycerintricaprinat und/oder Glycerintrilaurat verwendet.

3. Verfahren nach Anspruch 1 oder 2, dadurch gekennzeichnet, dass die genannte Atmosphäre bis zum Einfüllen der Mischung in Weichgelatinekapseln aufrecht erhalten wird.

**Claims**

1. Process for stabilizing of 4-methyl-5-(2'-chloroethyl)-thiazol by means of non toxic glycerides as carrier substance, for filling capsules, characterized in that one uses glycerides of saturated fatty acids having 8 to 12 carbon atoms, in an amount of 20 to 80 % by weight with respect to the whole mixture, and in that the mixing is carried out in the presence of less than 0.001 % of heavy metal cations and under exclusion of air oxygen in an inert gas atmosphere, especially under nitrogen.

2. Process according to claim 1, characterized in that the glycerides used are glycerine tricaprinate and/or glycerine trilaurate.

3. Process according to claim 1 or 2, characterized in that said atmosphere is maintained until filling the mixture into capsules of soft gelatine.

**Revendications**

1. Procédé pour stabiliser le 4-méthyl-5-(2'-chloreéthyl)-thiazole au moyen de glycérides non toxiques comme substance porteuse, pour remplir des capsules, caractérisé en ce qu'on utilise des glycérides d'acides gras saturés avec 8 à 12 atomes de carbone dans une quantité de 20 à 80 % en poids par rapport au poids total du mélange, et en ce que le mélange se fait en présence de moins de 0,001 % de cations de métaux lourds et à l'exclusion de l'oxygène de l'air dans une atmosphère de gaz inerte, notamment sous azote.

2. Procédé selon la revendication 1, caractérisé en ce qu'on utilise comme glycérides le tricaprinate de glycérine et/ou le trilaurate de glycérine.

3. Procédé selon la revendication 1 ou 2, caractérisé en ce que ladite atmosphère est maintenue jusqu'au remplissage du mélange des capsules en gélatine douce.